# NEUE EUROPÄISCHE PATENTSCHRIFT

(11) **EP 2 597 948 B2**
(45) Veröffentlichungstag und Bekanntmachung des Hinweises auf die Entscheidung über den Einspruch: **09.05.2018**
(45) Hinweis auf die Patenterteilung: 04.03.2015
(21) Anmeldenummer: 11738672.2
(22) Anmeldetag: 12.07.2011
(51) Int. Cl.: A01N 25/24, A01N 57/12, C11D 1/34

(54) **HAFTENDES SAURES SANITÄRREINIGUNGS- UND BEDUFTUNGSMITTEL**
ADHESIVE ACIDIC SANITARY CLEANING AND DEODORISING PRODUCT
AGENT ADHESIF ET ACIDE DE NETTOYAGE ET DE DESODORISATION POUR LES TOILETTES

(30) Priorität: 27.07.2010 DE 102010032417
(43) Veröffentlichungstag der Anmeldung: 05.06.2013
(73) Patentinhaber: Buck-Chemie GmbH, 71083 Herrenberg (DE)
(72) Erfinder: LEIPOLD, Joachim, 72760 Reutlingen (DE); JAESCHKE, Edgar, 70794 Filderstadt (DE); FRITZ, Matthias, 72810 Gomaringen (DE)
(74) Vertreter: Mammel und Maser
(86) Internationale Anmeldenummer: PCT/EP2011/061845
(87) Internationale Veröffentlichungsnummer: WO 2012/013490

(56) Entgegenhaltungen:
- WO-A1-97/40133
- WO-A1-99/66017
- WO-A1-2009/106220
- WO-A2-2004/067194
- DE-A1- 19 623 571
- DE-A1-102004 056 554
- DE-A1-102008 051 173
- GB-A- 2 397 823
- US-A- 5 334 325

## Beschreibung

Die Erfindung betrifft haftende Sanitäreinigungs- und Beduftungsmittel zum Reinigen und/oder Desinfizieren und/oder zur Duftstoffabgabe für Sanitärgegenstände wie Toilettenspülbecken, die sich auch zur Entfernung von Kalk und Urinstein eignen.

Solche haftenden Sanitärmittel sind viskose, im Allgemeinen pastöse Gele, die aus einem entsprechenden Behältnis direkt auf der Oberfläche des Sanitärgegenstandes aufgebracht werden, dort haften und erst nach einer größeren Anzahl von Spülvorgängen abspülbar sind. Im Allgemeinen sind diese Mittel wenigstens teilweise flüssigkristallin.

Durch die direkte Haftung des Mittels auf der Oberfläche des Sanitärgegenstandes ist es nicht erforderlich, zusätzlich Behältnisse wie die sogenannten "WC-Körbchen" vorzusehen, deren Benutzung vom Verbraucher insbesondere beim Ersetzen des Sanitärmittels und beim Putzen der Toilette als unhygienisch empfunden wird.

Solche haftenden Sanitärmittel sind aus der WO 99/66017 bekannt. Sie umfassen neben Tensiden, Wasser und Duftstoffen auch Haftvermittler, beispielsweise aus der Klasse der nichtionischen Tenside wie den Polyalkoxyalkanen und sind viskose, feste oder pastöse Mittel. Eine polarisationsmikroskopische Untersuchung der aus dieser Druckschrift bekannten Reinigungsmittel zeigt, dass die Mittel flüssigkristalline Phasen, insbesondere aus hexagonalen Strukturen, umfassen.

Um ein Austrocknen der Oberfläche dieser haftenden Mittel zu verhindern und eine unabhängig von der Anzahl der Spülungen ansehnliche und glatte Oberfläche bereitzustellen, wird in der DE 100 48 887 A1 vorgeschlagen, dem Mittel zusätzlich eine aliphatische Di-, Oligo- oder Polyhydroxyverbindung oder deren Ether zuzusetzen.

Aus der EP 1 318 191 ist ein Sanitärmittel bekannt, das als Haftvermittler Verbindungen aus der Gruppe der Oligo- oder Polyethylenoxid und/oder Oligo- und/oder Polypropylenoxid und/oder Oligo- und/oder Polybutylenoxid umfassenden Blockpolymere, der Arylethoxylate oder der Alkyl-Arylethoxylate umfasst.

Diese bekannten haftenden Sanitärmittel lassen sich auf einfache und hygienische Art und Weise mit einer geeigneten Vorrichtung applizieren, haften an der Oberfläche des Sanitärgegenstands, behalten ihre Form bei und werden selbst unter Einwirkung von Wasser nicht als Ganzes herabgespült, sondern lösen sich erst nach einer Vielzahl von Spülungen nach und nach vollständig auf.

Weiterhin sind aus der DE 103 56 254 A1 haftende Sanitärmittel bekannt geworden, die anionische Tenside und nichtionische Tenside als Haftvermittler und Silikate als Verdicker umfassen.

Diese Mittel zeichnen sich durch gute Handhabbarkeit und eine gute Reinigungswirkung aus. Durch die direkte Applizierbarkeit des Mittels ist es nicht mehr erforderlich, dass der Verbraucher beim Nachfüllen ein möglicherweise verunreinigtes Toilettenkörbchen berühren muss. Auch kann der Verbraucher das Mittel in der von ihm gewünschten Menge aufbringen und ist nicht auf die vom Hersteller vorgesehene Packungsmenge beschränkt.

Ein weiterer Vorteil der bekannten gelförmigen haftenden Mittel ist, dass sich diese durch eine im Vergleich zu den Reinigungsmittelformkörpern deutlich bessere Duftfreisetzung auszeichnen.

Eine Vielzahl der bislang bekannten haftenden Sanitärmittel ist gelförmig. Aufgrund der Instabilität der Gelstruktur führt allerdings der Zusatz von ansonsten bei Sanitärmitteln üblichen Zusatzstoffen zu diesen Gelen häufig zur Zerstörung der Gelstruktur.

In der DE 10 2004 056 554 A1 ist eine Weiterentwicklung solcher haftenden Gele, die trotz Bleichmittelzugabe beständig sind, beschrieben.

Aus der WO 2009/106220 A1 ist eine Abwandlung der haftenden Mittel auf der Grundlage weiterer Klassen von speziellen Haftvermittlern bekannt, mit denen es gelungen ist, eine derart klebrige Masse bereitzustellen, dass diese zur Befestigung von stückförmigen Mitteln in der Toilettenschüssel dienen kann, d.h. sozusagen als "Klebemittel" für Rimblocks, Duftsteine oder Entkalkertabletten in der Toilette fungiert und sich dennoch nach und nach abspült.

Die bislang bekannten haftenden Sanitärmittel sind neutral oder alkalisch und somit nicht wie die sauren Reiniger zur Entfernung von Kalk und Urinstein einsetzbar.

Der Zusatz der üblichen in Sanitärmitteln enthaltenen Säuren wie Amidosulfonsäure zu diesen haftenden meist gelförmigen Sanitärmitteln führt zur Zerstörung der Gelstruktur.

Aus der DE 19916182 A1 sind wässrige Zubereitungen für die Reinigung harter Oberflächen bekannt, die unter anderem Alkylaryletherphosphate enthalten. Die US 4,752,411 betrifft alkalische flüssige Reinigungsmittelzusammensetzungen, die unter anderem Phosphorsäureester enthalten. Aus der US 3,655,569 sind Mittel zur Reinigung harter Oberflächen bekannt, die unter anderem Phosphatester enthalten. Die US 3,432,494 und US 3,432,495 lehren Arylalkylarsensäuren und die US 2010/0158967 A1 lehrt biozite Formulierungen auf der Basis von Selenverbindungen.

Aus der DE 196 23 571 A1 ist ein Verdickungsmittel für wässrige Wasserstoffperoxidlösungen bekannt. Die WO 97/40133 A1 beschreibt sanitäre Reinigungsprodukte zur Entfernung von Verkalkungen, welche versprüht werden und auf Oberflächen haften. Aus der GB 2 397 823 A sind stabilisierte, wässrige Reinigungsmittel auf der Basis von Wasserstoffperoxid bekannt. Die DE 10 2008 051173 A1 offenbart ein haftendes Mittel für den Sanitärbereich, das als Haftvermittler unter anderem auch Polyalkylenimine oder Polyetheramine umfassen kann.

Die Aufgabe der vorliegenden Erfindung besteht darin, ein haftendes Sanitärmittel mit einer guten Reinigungsleistung bereitzustellen, mittels dessen auch Kalk und Urinstein wirksam entfernt werden können.

Diese Aufgabe wird durch Sanitärmittel gelöst, die als Haftvermittler ein saures Salz einer Oxosäure der folgenden Formel umfassen: 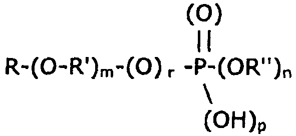 wobei R Alkyl- oder Arylreste, R' eine Alkyl oder eine Hydroxyalkylgruppe, R" ein Alkyl-, Aryl- oder Alkoxyalkylrest, m = 1 bis 50, n = 0,1 r = 0,1 und p = 1,2 ist und wobei die Viskosität des Mittels, gemessen mit einem Haake-Viskosimeter, System Platte-Kegel, Sensor PK 5 1°, Schergefälle von 25 s⁻¹ und 20 °C wenigstens 15.000 mPas beträgt.

Dadurch, dass der erfindungsgemäße Haftvermittler sauer ist, ist er gegen Urinstein und Kalk wirksam. Zudem zeigt er ein gutes Haftvermögen auf harten Oberflächen, die nass oder trocken sein können (Keramik, Fliesen, Metall, Glas, Kacheln etc.), so dass die den erfindungsgemäßen Haftvermittler umfassenden Mittel auch die gewünschte Haftung aufweisen und nach dem Ankleben erst nach und nach wieder abgespült werden. Weiterhin weist er die Eigenschaften eines anionischen Tensides auf.

Mit dem erfindungsgemäßen sauren Haftvermittler wird - anders als bei den herkömmlichen Säuren wie Amidosulfonsäure - die Gelstruktur bei einem gelförmigen System nicht zerstört.

Unter Oxosäuren werden im Rahmen der vorliegenden Erfindung alle sauerstoffhaltigen Säuren mit einem anorganischen Zentralatom verstanden.

Die Oxosäuren sind mit einem organischen Rest verestert, der einen endständige Alkyl- oder Arylrest R und eine Oligo- oder Polyethergruppe aufweist. Die Oligo- oder Polyethergruppe (OR') ist eine Oligo- oder Polyalkoxygruppe, die auch hydroxyliert sein kann, vorzugsweise eine Alkoxy- oder Hydroxyalkoxygrupe mit weniger als 8 Kohlenstoffatomen, besonders bevorzugt eine C2 und C3, d.h. die Oligoethergruppe OR' ist eine Ethoxy, Propoxy oder Glycerylgruppe (O-C-C(H)(OH)-C-).

Der sich an die Etherfunktion anschließende endständige Rest R ist eine Alkylgruppe oder eine Arylgruppe, vorzugsweise ein C₈-C₂₂-Alkylrest (verzweigt oder unverzweigt) und ein Benzyl(aryl)rest, aber auch ein Heteroaromat.

Sofern die freie Oxosäure neben der mit dem Oligo/Polyetherrest veresterten OH-Gruppe und der freien OH-Gruppe noch weitere OH-Gruppen aufweisen sollte, können die weiteren OH-Gruppen ebenfalls verestert sein, sofern die Verbindung noch sauer regiert.

In der Variante mit r=0 ist der Alkylrest R' direkt an das Zentralatom gebunden und somit nicht über eine weitere Sauerstoffbrücke verbrückt. Diese Verbindungsklasse ist ebenfalls möglich, solange diese die erforderliche Acidität aufweisen.

Der Anteil der erfindungsgemäßen sauren Haftvermittler in dem Mittel sollte zwischen 1 Gew.% und 50 Gew.%, vorzugsweise zwischen 3 Gew.% und 30 Gew.% und besonders bevorzugt zwischen 5 Gew.% und 26 Gew.% betragen.

Als erfindungsgemäße Haftvermittler können beispielsweise saure Alkylpolyglykoletherphosphate eingesetzt werden.

Besonders bevorzugte Haftvermittler sind die sauren Alkylpolyglykoletherphosphate, die beispielsweise unter dem Handelsnamen Phosfetal von Zschimmer & Schwarz, Lahnstein, Deutschland, Crafol von der Firma Cognis oder Naxonac von der Firma Nease Performance Chemicals erhältlich sind.

Diese sauren anionischen Tenside sind als Phosphorsäureester weniger empfindlich gegenüber Härtebildnern des Wassers wie Calcium oder Magnesium, besitzen ein gutes Kalkseifendispergiervermögen, sind sehr beständig gegenüber Alkalien und mit anionischen, amphoteren und nichtionischen Tensiden verträglich. Weiterhin sind sie korrosionsschützend, vollständig biologisch abbaubar und entsprechen der Detergenzienverordnung der EG Nr. 648/2004.

Bei einem Anteil von ca. 25 Gew.% der besonders bevorzugten sauren Alkylpolyglykoletherphosphate in dem Mittel kann ein pH-Wert von ca. 3,9 erreicht werden (0,1 % Lösung), bei einem Anteil von nur etwa 5 % steigt der pH-Wert des Mittels auf 6,75 (0,1% Lösung). Um den in dem erfindungsgemäßen Mittel gewünschten niederen pH-Wert zu erhalten sollte der Anteil an sauren Alkylpolyglykoletherphosphate in dem Mittel somit wenigstens 18 Gew.% und vorzugsweise 25 Gew.% betragen.

Alkylpolyglykoletherphosphat bildet zusammen mit Wasser eine harte klebende saure weiße Masse, die klebrig ist und sich nach und nach abspült.

Ein nur das Alkylpolyglyceroletherphosphate umfassendes Mittel ist sauer und zeigt die gewünschte Reinigungswirkung gegenüber Kalk und Urinstein, allerdings sind die Oberflächenspannung und das Schaumvermögen eines solchen Mittels gering.

Zur Verbesserung des Schaumvermögens und zur Absenkung der Oberflächenspannung können dem erfindungsgemäßen Mittel weitere Tenside zugesetzt werden. In einer bevorzugten Ausführungsform weist das erfindungsgemäße Mittel zur Verbesserung des Schaumvermögens anionische Fettalkoholethersulfate auf, die insbesondere aus der Gruppe der Ammoniumfettalkoholethersulfate mit vorzugsweise C₁₂-C₁₈-Alkylresten und 1 bis 6 EO ausgewählt werden. Ein besonders bevorzugtes anionisches Fettalkoholethersulfat ist Triisopropanolammoniumfettalkoholethersulfat mit 1,2 Propylenglykol, das beispielsweise bei Zschimmer & Schwarz unter dem Handelsnamen Zetesol TP 300 erhältlich ist.

Dieses anionische Tensid zeichnet sich durch ein sehr hohes Schaumvermögen und eine niedrige Oberflächenspannung aus, es reagiert in wässriger Lösung neutral, haftet in Kombination mit Wasser ebenfalls gut an der Oberfläche und lässt sich nach und nach abspülen.

Der Anteil an diesen anionischen Fettalkoholethersulfaten sollte in dem Mittel bis zu 60 Gew.%, vorzugweise zwischen 10 Gew.% und 40 Gew.% und besonders bevorzugt zwischen 15 Gew.% und 25 Gew.% liegen.

Im Rahmen der vorliegenden Erfindung wurde festgestellt, dass bei der gemeinsamen Verwendung des sauren erfindungsgemäßen Haftermittlers mit dem anionischen Fettalkoholethersulfat ein saures, gut klebendes, erst nach einer Vielzahl von Spülvorgängen nach und nach abspülbares Mittel erhalten wird, das zudem gut schäumt und gut reinigt, da es eine niedrige Oberflächenspannung aufweist. Zudem lässt sich der gewünschte saure pH-Wert des Mittels einstellen und die sauren Eigenschaften des Mittels somit an die erforderliche Entkalkungsleistung anpassen. Ein weiterer Vorteil dieser Kombination der beiden Tenside besteht darin, dass beide an der nassen und trockenen Oberfläche die gewünschte Klebewirkung entfalten.

Vorzugsweise beträgt das Verhältnis der sauren Alkylpolyglykoletherphosphate zu anionischem Fettalkoholethersulfat zwischen 1:100 und 100:1, vorzugsweise 1:10 und 10:1.

Selbstverständlich ist es möglich, anstelle des anionischen Fettalkoholethersulfats auch ein anderes gut schäumendes und gut reinigendes Tensid zuzusetzen wie beispielsweise amphotere Tenside.

Das erfindungsgemäße Mittel kann weiterhin andere Tenside, Lösemittel, Duftstoffe, Farbstoffe, Desinfektionsmittel, Konservierungsstoffe wie zum Beispiel Isothiazolon-Derivate oder Schaumstabilisatoren wie Kokosfettsäuremono/Diethanolamid oder Alkylaminoxide umfassen, sofern die Zusätze unter sauren Bedingungen stabil sind.

Ein weiterer Vorteil des erfindungsgemäßen Mittels ist, dass es ein hohes Parfümaufnahmevermögen besitzt und einen Parfümanteil von größer gleich 8 % aufweisen kann. Das Mittel umfasst gewöhnlich zwischen 2 Gew.% und 15 Gew.% und bevorzugt zwischen 4 Gew.% und 10 Gew.% Parfümöl.

Die erfindungsgemäßen Haftvermittler wie beispielsweise das bevorzugte anionische Alkylpolyglycolethersulfat bilden bei Zugabe von Wasser eine knetmassenähnliche Substanz, die mit zunehmendem Wassergehalt immer stärker eindickt, dann ein Viskositätsmaximum erreicht. Nach diesem Maximum nimmt die Viskosität bei Zugabe von weiterem Wasser wieder ab.

Die erfindungsgemäßen Rezepturen sind vorzugsweise so eingestellt, dass die Masse das Viskositätsmaximum noch nicht erreicht hat, d. h., dass die z.B. an der Toilettenschüssel befindliche Masse beim ersten Überspülen weiter eindickt und sich verfestigt.

Die für die Applikation ggf. erforderliche geringere Viskosität des Mittels kann beispielsweise durch Zugabe von polaren Lösemitteln wie Glycerin, Alkoholen wie Methanol oder Ethanol, aber auch unpolaren Lösemitteln wie den typischen Parfümlösemitteln oder auch Parfümöl selbst eingestellt werden. Je höher der Anteil an nicht wässrigen Lösemitteln in einer Masse, desto flüssiger wird das Mittel.

Die Konzentration von Wasser liegt im Allgemeinen zwischen 20 Gew.% und 80 Gew.%, vorzugsweise zwischen 30 Gew.% und 60 Gew.% und besonders bevorzugt zwischen 40 Gew.% und 50 Gew.%.

Der Anteil an nichtwässrigen Lösemitteln (einschließlich Parfümöl) in dem Mittel liegt im Allgemeinen zwischen 0,2 Gew.% und 20 Gew.%, vorzugsweise zwischen 1 Gew.% und 15 Gew.% und besonders bevorzugt zwischen 2 Gew.% und 14 Gew.%.

Das Verhältnis Tensid : Wasser beträgt vorzugsweise zwischen 0,9:1 und 1,2:1, und das Verhältnis Tensid : Gesamtlösemittel (einschließlich Parfümöl) liegt vorzugsweise zwischen 0,7:1 und 1,1:1.

Als weitere Tenside können prinzipiell alle bekannten anionischen und kationischen oder amphoteren Tenside eingesetzt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alkan- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht.

Geeignet sind auch Alkansulfonate, die aus C₁₂₋₁₈-Alkanen, beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monogylcerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Auch 2,3-Alkylsulfate, welche als Handelsprodukte der Shell Oil Company unter dem Namen DAN® erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxilierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Die anionischen Tenside können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze, vor.

Als anionische Tenside werden erfindungsgemäß besonders bevorzugt Fettalkoholethersulfate, Alkylsulfate, Alkylbenzolsulfonate und/oder Alkansulfonate eingesetzt.

Bei den fakultativ enthaltenen zusätzlichen Parfümstoffen handelt es sich um die aus dem Stand der Technik gängigen. Als Beispiele seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind zum Beispiel Benzylacetat, Phenoxyethylisobutyrat, p-ter.-Butylcyclohesyl-acetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylalicylat. Zu den Ethern zählen beispielsweise Benzylethylether, zu den Aldehyden zum Beispiel die linearen Alkanale mit 8 bis 18 Kohlenstoffatomen, Citral, Citronellal, Citronellyloxyacetaldehyd, Cylamenaldehyd, Hydroxycitronellal, Lilial und Bourgeonal, zu den Ketonen zum Beispiel die Jonone, α-Isomethylionen und Methylcedrylketon, zu den Terpenalkoholen Menthol, Anethol, Citronellol, Eugenol, Isoeugenol, Geraniol, Linalool, Nerol, Phenylethylalkohol, Tetrahydromyrcenol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Auch ätherische Öle geringerer Flüchtigkeit, die meist als Aromakomponenten verwendet werden, eignen sich als Parfümöle, zum Beispiel Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandilöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller, Salbeiöl, β-Damascone, Geraniumöl Bourbon, Cyclohexylsalicylat, Vertofix Coeur, Iso-E-Super, Fixolide NP, Evernyl, Iraldein gamme, Phenylessigsäure, Geranylacetat, Benzylacetat, Rosenoxid, Romillat, Irotyl und Floramat allein oder in Mischungen, eingesetzt.

Die Menge der Dosierung ist abhängig von der gewünschten Duftintensität und liegt vorzugsweise im Bereich von 5 Gew.% - 20 Gew.%, besonders bevorzugt von 6 Gew.% bis 8 Gew.% Besonders bevorzugt sind die Duftstoffe säurestabil.

Auch ist es möglich, dem erfindungsgemäßen Mittel Olefinsulfonate, Ethersulfate und/oder Tenside, insbesondere Säuremethyltauride als Schäumer mit Selbstreinigungswirkung zuzusetzen.

Um das Austrocknen des Mittel zu verhindern, kann das Mittel zusätzlich noch vorzugsweise zwischen 5 und 15 Gew.%, aliphatische Di-, Oligo- oder Polyhydroxyverbindungen oder deren Ether oder der Verbindung(en) aus der Gruppe Glycerin, 1,3-Dihydroxypropan, 1,3- oder 1,4-Dihydroxybutan, 1,3-Dihydroxyisobutan und/oder Pentaerythrit umfassen.

Der Formulierung können - falls gewünscht - auch Salze, wie beispielsweise Natriumsulfat, hinzugegeben werden, um die

Auflösegeschwindigkeit zu erhöhen. Der Salzanteil kann bis zu 10 Gew.%, vorzugsweise bis zu 5 Gew.%, betragen.

Das erfindungsgemäße Mittel kann in hygienischer Weise ohne Berührung von mit dem WC-Becken verbundenen, möglicherweise verunreinigten Vorrichtungen aufgebracht und erneuert werden.

Ein wesentlicher Vorteil des erfindungsgemäßen Mittels besteht darin, dass es nach den Wünschen des Verbrauchers portionierbar ist. Wünscht der Verbraucher eine intensivere Reinigung oder wird die Toilette häufig benutzt, so kann entsprechend stärker dosiert werden.

Das erfindungsgemäße Mittel kann auch auf einfache Weise gleichzeitig an verschiedenen Stellen des Sanitärgegenstands appliziert werden, beispielsweise, um sowohl auf der rechten, als auch auf der linken Seite einer Toilettenschüssel eine gleichmäßige Reinigungswirkung zu erzielen.

Die erreichte Haftung ist an dem Sanitärgegenstand selbst bei einer Anbringung an einer senkrechten Fläche so gut, dass sich das Mittel sogar unter der zusätzlichen Krafteinwirkung von Spülwasserströmen nicht ablöst.

Die erfindungsgemäßen Sanitärmittel sind erst nach einer größeren Anzahl von Spülvorgängen abspülbar. Die Anzahl der Spülvorgänge richtet sich natürlich nach der Zusammensetzung des jeweiligen Sanitärmittels, der aufgebrachten Menge sowie der Geometrie des aufgebrachten Sanitärmittels.

Vorzugsweise ist das erfindungsgemäße Mittel eine salbenartige pastöse, cremeartige oder gelförmige Masse, wobei die gelförmige Masse insbesondere flüssigkristallin ist und bevorzugt eine hexagonale Struktur aufweist. Die Gele sind im Wesentlichen formstabil, so dass sie nicht "herunterlaufen" oder "tropfen".

Die Gele lassen sich vorzugsweise über Tuben, vergleichbar den Zahnpastatuben, Spritzen oder Kartuschen in das Toilettenbecken einbringen und bleiben dort für eine Vielzahl von aufeinanderfolgenden Spülvorgängen haften.

Die an einem Haake-Viskosimeter, System Platte-Kegel, Sensor PK 5 1°, Schergefälle von 25 s⁻¹ und 20°C zu bestimmenden Viskositäten dieser pastösen Gele betragen wenigstens 15.000 mPas. Üblicherweise sollten sie wenigstens 50.000 mPas, vorzugsweise wenigstens 90.000 mPas und besonders bevorzugt wenigstens 110.000 mPas betragen.

Die Spülzahlen des erfindungsgemäßen Mittels liegen beim Auftrag eines Streifens von 4 - 6 cm Länge, 2 cm Breite und 0,3 - 0,5 cm Dicke zwischen etwa zwischen 40 und 180.

Die erfindungsgemäßen Mittel sind vorzugsweise transparent.

Die Erfindung wird nachfolgend anhand von Ausführungsbeispielen näher beschrieben.

### 1. Herstellung des erfindungsgemäßen Mittels

Zur Herstellung der erfindungsgemäßen Mittel wird Wasser vorgelegt auf eine Temperatur T > 80 °C erwärmt und der erfindungsgemäße saure Haftvermittler und ggf. weiteres Tensid und Lösemittel zugegeben und gerührt. Man erhält eine knetfähige Masse. Bei der Zugabe des Phosfetals "stockt" die Masse und weist eine fast wachsartige Konsistenz auf.

Anschließend wird nach leichtem Abkühlen das wärmempfindliche Parfüm, das vorzugsweise säurestabil sein sollte, zugegeben und die Oberflächenspannung und die Spülzahlen bestimmt.

Falls gewünscht kann bei der Herstellung auch ein Farbstoff, beispielsweise ein 1% wässrige Rhodamin EB4-Lösung, zugegeben werden.

Um die Transparenz des Mittels zu erhöhen und den Einschluss von Luftblasen zu vermeiden sollte die Masse entgast werden.

Die gewünschte Schaumzahl und Oberflächenspannung können über die Menge des Fettalkoholethersulfats (wie z.B. Zetesol) eingestellt werden.

Die Verdickung in dem System Phosfetal/Wasser nimmt bei der Zugabe von Wasser zunächst zu, die Verdickung weist ein Maximum auf und nimmt dann bei weiterer Zugabe von Wasser wieder ab.

Die Konsistenz des Mittels kann durch Zugabe von Wasser oder anderen Lösemitteln wie beispielsweise polaren Lösemitten (Alkohole, PEG 300, PEG 400) oder auch Parfüm eingestellt werden.

Bei der Herstellung wird die Masse so eingestellt, dass das Viskositätsmaximum noch nicht erreicht ist.

Die Masse verdickt nach der Herstellung nach Zugabe von weiterem Wasser nach (vergelt weiter). Um die verdickte Masse aus einem Applikator auftragen zu können, wird die einzufüllende Masse mit nichtwässrigem Lösemittel wie Alkohol oder Parfüm (Stellmittel) verdünnt. Beim ersten Überspülen der Masse in der Toilettenschüssel wird nichtwässriges Lösemittel sukzessive durch Wasser ersetzt. Dadurch dickt die Masse bis in das Viskositätsmaximum nach.

Die erhaltene Masse kann mit einem normalen Applikator auf eine trockene oder feuchte Oberfläche appliziert werden, haftet dort und ist erst nach einer größeren Anzahl von Spülvorgängen abspülbar.

Die so erhaltene Masse ist sauer, d.h. sie löst Kalk und Urinstein, und ist ein transparentes Gel.

### 2. Rezepturen

In **Tabelle I** sind verschiedene Rezepturen der hergestellten Mittel zusammengestellt, die Rezepturen 2, 3, 4, 6 und 7 sind erfindungsgemäß. Auch enthält diese Tabelle Angaben über die Konsistenz, das Klebeverhalten, die Spülzahl, den pH-Wert, die Oberflächenspannung und die Schaumzahlen der jeweiligen Mittel.

Als saurer Haftvermittler wurde das anionische Tenside Phosfetal 205, erhältlich bei Zschimmer & Schwarz, eingesetzt. Der pH-Wert einer 1% Lösung beträgt 2. Die Alkylgruppe ist eine C14- C18-Gruppe, die "Polyglykolether"-Gruppe weist zwischen 1 und 3 Glykoleinheiten auf.

Als anionisches Fettalkoholethersulfat wurde Triisopropanolammoniumfettalkoholethersulfat mit 1,2-Propylenglykol (TIPA-Laureth Sulfate (and) Propylene Glycol) eingesetzt, das bei Zschimmer & Schwarz unter dem Handelsnamen Zetesol TP 300 erhältlich ist. Der Fettalkoholrest weist zwischen 12 und 14 Kohlenstoffatome auf und ist mit 2,5 - 3 EO ethoxyliert. Die 2% wässrige Lösung dieses Tensids reagiert neutral (pH 7).

Als Parfüm wurde Orange Fun, erhältlich bei der Firma Givaudan, eingesetzt.

### 3. Bestimmung der Schaumzahlen und der Oberflächenspannung

Zur Bestimmung der Schaumzahlen überführt man 100 ml der auf 20 °C temperierten Stammlösung in einen 250 ml Mischzylinder und verschließt mit einem PTFE-Stopfen. Dann wird der Zylinder zwanzigmal hin und her geschwenkt (20mal auf den Kopf gestellt). Nach jeweils 30 sec, 5 Minuten und 30 Minuten wir das erzeugte Schaumvolumen (ml) abgelesen und notiert.

Die Bestimmung der Oberflächenspannung erfolgte mit dem Gerät BP2 der Fa. Krüss Blasendruckmessgeräte.

**Tabelle I:**

| **Versuch** | **1** | **2** | **3** | **4** | |
|---|---|---|---|---|---|
| | **[%]** | **[%]** | **[%]** | **[%]** | |
| **Zetesol TP 300** | 25 | 22,7 | 25 | 0 | |
| **Phosfetal 205** | 25 | 22,7 | 25 | 50 | |
| **Parfüm (Orange Fun)** | 8 | 7,27 | 5 | 5 | |
| **Wasser** | 42 | 47,3 | 45 | 45 | |
| **Summe** | 100 | 100 | 100 | 100 | |
| | | | | | |
| **Konsistenz** | viskos/ fließend | gestocktes Material | stockt unter Herstellung | stockt unter Herstellung | |
| **Spülzahl** | ./. | n.b. | > 187 | n.b. | |
| **pH, 0,1%ige Lösung** | | | 3,94 (21,3 °C) | | |
| **Klebt** | nein (fließt) | ja | ja | ja | |
| **Oberflächenspannung [mN/m]** | | | 67,2/59,1/54, 9 | 70,1; 6,3; 65,3 | |
| **Schaum [30s, 5min., 30min.]** | | | 115/105/90 | 20; 15; 10 | |
| **Summe Tensid** | 50 | 50 | 50 | 50 | |
| **Tensid: Wasser** | 1,19 | 0,96 | 1,11 | 1,11 | |
| **Tensid: Gesamtlösemittel** | 1 | 0,83 | 1 | 1 | |

| **Versuch** | **5** | **6** | **7** | **8** | **Hersteller** |
|---|---|---|---|---|---|
| | **[%]** | **[%]** | **[%]** | **[%]** | |
| **Zetesol TP 300** | 50 | 44,61 | 5,00 | 20 | Zschimmer & Schwarz |
| **Phosfetal 205** | 0 | 5,71 | 44,97 | 20 | Zschimmer & Schwarz |
| **Parfüm (Orange Fun)** | 5 | 4,95 | 5,06 | 20 | Givaudan |
| **Wasser** | 45 | 44,72 | 44,97 | 40 | Demineralisiert |
| **Summe** | 100 | 100 | 100 | 100 | |
| | | | | | |
| **Konsistenz** | stockt unter Herstellung | stockt unter Herstellung | stockt unter Herstellung | Masse zu dünn | |
| **Spülzahl** | > 35 | n.b. | n.b. | ./. | |
| **pH, 0,1%ige Lösung** | | 6,75; (27,3°C) | | | |
| **Klebt** | Ja | ja | ja | klebt nicht | |
| **Oberflächenspannung [mN/m]** | 57,5; 45,9; 42,1 | 60/49,1/45,1 | | | |
| **Schaum [30s, 5min., 30min.]** | 190; 170; 160 | 125/110/100 | | | |
| **Summe Tensid** | 50 | 51,1 | 50 | 40 | |
| **Tensid: Wasser** | 1,11 | 1,13 | 1,11 | 1 | |
| **Tensid: Gesamtlösemittel** | 1 | 1,01 | 1,00 | 0,67 | |

## Patentansprüche

1. Sanitärmittel zum Reinigen und/oder Desinfizieren und/oder zur Duftstoffabgabe, welches Mittel unmittelbar auf den Sanitärgegenstand applizierbar ist, dort haftet und erst nach einer größeren Anzahl von Spülvorgängen abspülbar ist und welches Mittel Tenside, Lösemittel und wenigstens einen Haftvermittler umfasst, **dadurch gekennzeichnet, dass** der Haftvermittler eine Oxosäure der nachfolgenden Formel 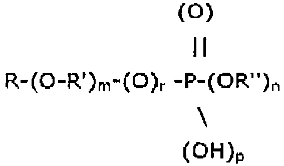 ist und R ein Alkyl- oder Arylreste, R' eine Alkyl- oder Hydroxyalkylgruppe, R" ein Alkyl-, Aryl oder Alkoxyalkylrest, m = 1 bis 50, n = 0, 1, r = 0, 1 und p = 1, 2 ist und die Viskosität des Mittels, gemessen mit einem Haake-Viskosimeter, System Platte-Kegel, Sensor PK 5 1°, Schergefälle von 25 s⁻¹ und 20 °C wenigstens 15.000 mPas beträgt.

2. Sanitärmittel nach Anspruch 1, **dadurch gekennzeichnet, dass** der Haftvermittler ein Alkylpolyglykoletherphosphat ist.

3. Sanitärmittel nach Anspruch 1, **dadurch gekennzeichnet, dass** der Anteil des Haftvermittlers in dem Mittel zwischen 1 Gew.% und 50 Gew.% beträgt.

4. Sanitärmittel nach Anspruch 3, **dadurch gekennzeichnet, dass** der Anteil des Haftvermittlers in dem Mittel zwischen 3 Gew.% und 30 Gew.% beträgt.

5. Sanitärmittel nach einem der vorangegangenen Ansprüche, **dadurch gekennzeichnet, dass** es zusätzlich anionische und/oder nichtionische und/oder amphotere und/oder nichtionische Tenside umfasst.

6. Sanitärmittel nach einem der vorangegangenen Ansprüche, **dadurch gekennzeichnet, dass** das Mittel Fettalkoholethersulfate umfasst.

7. Sanitärmittel nach Anspruch 6, dass der Anteil an Fettalkoholethersulfaten bis zu 60 Gew.%, beträgt.

8. Sanitärmittel nach einem der vorangegangenen Ansprüche, **dadurch gekennzeichnet, dass** das Mittel Wasser in einer Konzentration zwischen 20 Gew.% und 80 Gew.% umfasst.

9. Sanitärmittel nach einem der vorangegangenen Ansprüche, **dadurch gekennzeichnet, dass** das Mittel Parfümöl umfasst.

10. Sanitärmittel nach Anspruch 9, **dadurch gekennzeichnet, dass** es Parfüm in einer Konzentration zwischen 2 Gew.% und 15 Gew.% umfasst.

11. Sanitärmittel nach einem der vorangegangenen Ansprüche, **dadurch gekennzeichnet, dass** die Spülzahlen des Mittels, bestimmt an einen auf einer Toilettenschüssel aufgetragenen Streifen mit 4 bis 6 cm Länge, 2 cm Breite und 0,3 bis 0,5 cm Dicke zwischen 40 und 180 liegen.

12. Sanitärmittel nach einem der vorangegangenen Ansprüche, **dadurch gekennzeichnet, dass** das Mittel transparent ist.

13. Sanitärmittel nach einem der vorangegangenen Ansprüche, **dadurch gekennzeichnet, dass** das Mittel gelförmig ist.

## Claims

1. Sanitary agent for cleaning and/or disinfecting and/or fragrancing, said agent being able to be applied directly to the sanitary object, adhering there and being able to be flushed away only after a large number of flushing operations, and said agent comprising surfactants, solvents and at least one adhesion promoter, **characterized in that** the adhesion promoter is an oxo acid of the following formula 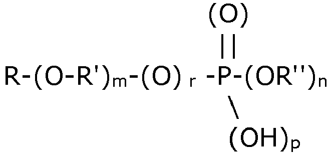 and R is an alkyl or aryl residue, R' is an alkyl or hydroxyalkyl group, R" is an alkyl, aryl or alkoxyalkyl residue, m = 1 to 50, n = 0, 1, r = 0, 1 and p = 1, 2, and the viscosity of the agent, measured with a Haake viscometer, plate-cone system, PK 5 1° sensor, shear rate of 25 s⁻¹ and 20°C, is at least 15 000 mPas.

2. Sanitary agent according to claim 1, **characterized in that** the adhesion promoter is an alkyl polyglycol ether phosphate.

3. Sanitary agent according to claim 1, **characterized in that** the proportion of the adhesion promoter in the agent is between 1% b.w. (by weight) and 50% b.w.

4. Sanitary agent according to claim 3, **characterized in that** the proportion of the adhesion promoter in the agent is between 3% b.w. and 30% b.w.

5. Sanitary agent according to one of the preceding claims, **characterized in that** it additionally comprises anionic and/or non-ionic and/or amphoteric surfactants.

6. Sanitary agent according to one of the preceding claims, **characterized in that** the agent comprises fatty alcohol ether sulfates.

7. Sanitary agent according to claim 6, **characterized in that** the proportion of fatty alcohol ether sulfates is up to 60% b.w.

8. Sanitary agent according to one of the preceding claims, **characterized in that** the agent comprises water in a concentration of between 20% b.w. and 80% b.w.

9. Sanitary agent according to one of the preceding claims, **characterized in that** the agent comprises perfume oil.

10. Sanitary agent according to claim 9, **characterized in that** it comprises perfume in a concentration of between 2% b.w. and 15% b.w.

11. Sanitary agent according to one of the preceding claims, **characterized in that** the flush numbers of the agent, determined on a strip of 4-6 cm in length, 2 cm in breadth and 0.3-0.5 cm in thickness, applied to a lavatory bowl, are between 40 and 180.

12. Sanitary agent according to one of the preceding claims, **characterized in that** the agent is transparent.

13. Sanitary agent according to one of the preceding claims, **characterized in that** the agent is gelous.

## Revendications

1. Agent sanitaire pour le nettoyage et/ou la désinfection et/ou pour la libération d'un parfum, ledit agent étant applicable directement sur l'article sanitaire, y adhérant et n'étant éliminé par rinçage qu'après un grand nombre de processus de rinçage, et ledit agent comprenant des tensioactifs, des solvants et au moins un promoteur d'adhésion, **caractérisé en ce que** le promoteur d'adhésion est un oxoacide de la formule suivante: 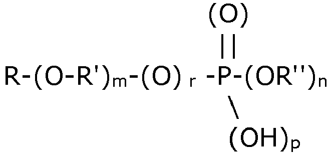 et R est un radical alkyle ou aryle, R' est un groupe alkyle ou hydroxyalkyle, R " est un radical alkyle, aryle ou alcoxyalkyle, m = 1 à 50, n = 0, 1, r = 0, 1 et p = 1, 2, et la viscosité de l'agent, mesurée avec un viscosimètre de Haake, un système à plaque et cône, un capteur PK 5 1°, un taux de cisaillement de 25 s⁻¹ et à 20 °C, est d'au moins 15 000 mPas.

2. Agent sanitaire selon la revendication 1, **caractérisé en ce que** le promoteur d'adhésion est un éther-phosphate d'alkyl-polyglycol.

3. Agent sanitaire selon la revendication 1, **caractérisé en ce que** la proportion du promoteur d'adhésion dans l'agent est comprise entre 1 % en poids et 50 % en poids.

4. Agent sanitaire selon la revendication 3, **caractérisé en ce que** la proportion du promoteur d'adhésion dans l'agent est comprise entre 3 % en poids de 30 % en poids.

5. Agent sanitaire selon l'une quelconque des revendications précédentes, **caractérisé en ce qu'**il comprend en outre des tensioactifs anioniques et/ou non ioniques et/ou amphotères.

6. Agent sanitaire selon l'une quelconque des revendications précédentes, **caractérisé en ce que** l'agent comprend des éther-sulfates d'alcools gras.

7. Agent sanitaire selon la revendication 6, **caractérisé en ce que** la proportion d'éther-sulfates d'alcools gras est de jusqu'à 60 % en poids.

8. Agent sanitaire selon l'une quelconque des revendications précédentes, **caractérisé en ce que** l'agent comprend de l'eau en une concentration comprise entre 20 % en poids et 80 % en poids.

9. Agent sanitaire selon l'une quelconque des revendications précédentes, **caractérisé en ce que** l'agent comprend une huile parfumée.

10. Agent sanitaire selon la revendication 9, **caractérisé en ce qu'**il comprend un parfum en une concentration comprise entre 2 % en poids et 15 % en poids.

11. Agent sanitaire selon l'une quelconque des revendications précédentes, **caractérisé en ce que** le nombre de rinçages de l'agent, déterminé par une bande d'une longueur de 4 à 6 cm, d'une largeur de 2 cm et d'une épaisseur de 0,3 à 0,5 cm, placée dans une cuvette de toilette, est compris entre 40 et 180.

12. Agent sanitaire selon l'une quelconque des revendications précédentes, **caractérisé en ce que** l'agent est transparent.

13. Agent sanitaire selon l'une quelconque des revendications précédentes, **caractérisé en ce que** l'agent est un gel.
